# EUROPEAN PATENT APPLICATION

(11) **EP 1 806 578 A1**
(43) Date of publication of application: **11.07.2007**
(21) Application number: 05787977.7
(22) Date of filing: 29.09.2005
(51) Int. Cl.: G01N 31/22, G01N 33/52

(54) **MULTILAYERED ANALYTICAL ELEMENT**

(30) Priority: 30.09.2004 JP 2004285852
(71) Applicant: FUJIFILM Corporation, Minato-ku Tokyo (JP)
(72) Inventor: NIRASAWA, Mitsuharu c/o FUJIFILM CORPORATION, Saitama 351-8585 (JP); KAGEYAMA, Shigeki c/o FUJIFILM CORPORATION, Saitama 351-8585 (JP); ITO, Toshihisa c/o FUJIFILM CORPORATION, Saitama 351-8585 (JP)
(74) Representative: Albrecht, Thomas
(86) International application number: PCT/JP2005/017953
(87) International publication number: WO 2006/035873

(57) **Abstract**

The object of the invention is to provide a multilayer analytical element having a non-fibrous porous film with high film strength, which does not produce uneven coloration in the form of a white-out and which has high accuracy of analysis. The present invention provides a dry multilayer analytical element for the analysis of a liquid sample which comprises a water-non-transmitting planar support on one side of which at least one adhesion layer and a porous liquid-sample-developing layer are integrally layered in the mentioned order, wherein the porous liquid-sample-developing layer comprises a non-fibrous porous film whose bending fracture strength is 20 g weight or more and whose elongation ratio when pulled with a 50-g weight is 2% or less, and wherein a adhesion layer polymer binder is caused to become seeped up in the non-fibrous porous film to 2 to 50 µm.

## Description

### TECHNICAL FIELD

The present invention relates to a dry multilayer analytical element used for clinical diagnoses, food inspection, environmental analytical and the like, and a method of producing the same.

### BACKGROUND ART

In the fields of clinical diagnoses, food inspection, and environmental examination, there is a growing demand for analyzing a specimen quickly and easily, and dry analytical elements are generally employed to meet such needs. In a dry analytical element, the developing layer, which is used for the reception, development and diffusion of blood or the like, has been typically formed of a fibrous porous material, as described in JP Patent Publication (Kokai) Nos. 55-164356 A (1980), 57-66359 A (1982), and 60-222769 A (1985), for example.

The fibrous porous material has a high spreading rate upon spotting of a liquid sample and is easy to handle during manufacture. It is also compatible with viscous samples, such as whole blood, and is therefore widely used.

In the relevant fields, increasingly higher measurement accuracies (reproducibility) are being required, and several inconveniences have been identified in the fibrous porous material (fabric developing layer). One of the inconveniencies relates to the problem of lot variations in the fabric. Normally, the fabric developing layer is available in woven material and knitted material, and lot-to-lot and intra-lot differences in the manner of weaving or knitting have been found. Specifically, the variations involve the number of stitches per unit area, the weight per unit area, and thickness, for example. There are also lot-to-lot and intra-lot differences in the hydrophilicity of the fabric depending on the degree of washing in the material-washing step in an intermediate process. Furthermore, as the fabric developing layer is not smooth, the developing layer must inevitably be wedged into the lower layer if a sufficient adhesive force is to be ensured by the laminating method during manufacturing. As a result, the lower layer is disturbed and is not suitable for analysis requiring high accuracy. The fabric also tends to extend when adhere to the lower layer for structural reasons, often resulting in a change in its gap volume. The change in the gap volume often leads to a change in the area of spreading of a liquid sample upon spotting, thus resulting in the intra-lot difference and preventing an accurate analysis. While there is a growing demand for analysis with smaller sample amounts, the fabric developing layer tends to have increasing variations in the amount of light it reflects as the amount of sample solution is reduced, due to the influence of its stitches. Furthermore, there is the problem that accurate analysis is prevented by the uneven disturbances introduced in the lower layer upon adhesion of the developing layer.

As a technique to replace the fabric developing layer, a method has been proposed whereby a porous film is produced by coating. A typical example is the so-called brush polymer layer (JP Patent Publication (Kokai) No. 49-53888 A (1974)) that takes advantage of the polymer phase transition reaction during coating/drying. Another example is a bead developing layer (JP Patent Publication (Kokai) No. 55-90859 A (1980)) that is formed by coating microbeads. These methods, however, have the disadvantage that the developing layer is weak and tends to become peeled when a sheet-like coated material is rendered into a slide (during processing).

In order to overcome the aforementioned problems, a method has been proposed whereby a pre-formed, homogeneous non-fibrous porous film having high film strength is laminated as a developing layer (JP Patent Publication (Kokai) No. 49-53888 A (1974); JP Patent Publication (Kokai) No. 56-96245 A (1981); and JP Patent Publication (Kokai) No. 56-97872 A (1981)). Typical methods for laminating such non-fibrous porous film are disclosed in JP Patent Publication (Kokai) No.60-222770 A (1985) and JP Patent Publication (Kokai) No. 7-26959 A (1995). These methods involve wetting the lower layer uniformly with water so as to cause a water-soluble polymer agent in the lower layer to seep up for bonding.

However, such non-fibrous porous film, when used for manufacturing a multilayer analytical element, has the disadvantage that the film strength of the film is too weak to provide processing suitability. Further, density unevenness often appears at the center in the form of a white-out upon coloration following spotting, which adversely affects the measurement reproducibility.

### DISCLOSURE OF THE INVENTION

### PROBLEMS TO BE SOLVED BY THE INVENTION

It is an object of the invention to solve the aforementioned problems of the background art. Specifically, the object of the invention is to provide a multilayer analytical element having a non-fibrous porous film with high film strength, which does not produce uneven coloration in the form of a white-out and which has high measurement accuracy.

### MEANS FOR SOLVING THE PROBLEMS

The present inventors have made an intensive research and analysis to solve the aforementioned objects, and have found that the aforementioned objects can be solved by employing a multilayer analytical element having a developing layer comprising a non-fibrous porous film whose bending fracture strength is 20 g weight or more and whose elongation ratio upon pulling with a 50-g weight is 2% or less, wherein a adhesion layer polymer binder is caused to become seeped up in the non-fibrous porous film to 2 to 50 µm. Thus, the present invention has been completed.

Specifically, the invention provides a dry multilayer analytical element for the analysis of a liquid sample which comprises a water-non-transmitting planar support on one side of which at least one adhesion layer and a porous liquid-sample-developing layer are integrally layered in the mentioned order, wherein the porous liquid-sample-developing layer comprises a non-fibrous porous film whose bending fracture strength is 20 g weight or more and whose elongation ratio when pulled with a 50-g weight is 2% or less, and wherein a adhesion layer binder is caused to become seeped up in the non-fibrous porous film to 2 to 50 µm.

Preferably, the adhesion layer polymer binder is caused to become seeped up in the non-fibrous porous film to 2 to 40 µm. More preferably, the adhesion layer polymer binder is caused to become seeped up in the non-fibrous porous film to 7 to 30 µm

Preferably, the non-fibrous porous film comprises: 6, 6-nylon; 6-nylon; acrylate copolymer; polyacrylate; polyacrylonitrile; polyacrylonitrile copolymer; polyamide, polyimide; polyamide-imide; polyurethane; polyether sulfone; polysulfone; a mixture of polyether sulfone and polysulfone; polyester; polyester carbonate; polyethylene; polyethylene chlorotrifluoroethylene copolymer; polyethylene tetrafluoroethylene copolymer; polyvinyl chloride; polyolefin; polycarbonate; polytetrafluoroethylene; polyvinylidene difluoride; polyphenylene sulfide; polyphenylene oxide; polyfluorocarbonate; polypropylene; polybenzoimidazole; polymethyl methacrylate; styrene-acrylonitrile copolymer; styrene-butadiene copolymer; a saponified substance of ethylene-vinyl acetate copolymer; polyvinyl alcohol; and a mixture thereof. More preferably, the non-fibrous porous film comprises polysulfone, polyether sulfone, 6,6-nylon, or 6-nylon.

Preferably, the non-fibrous porous film is an asymmetric film.

Preferably, the film thickness of the non-fibrous porous film is 80 to 300 µm.

Preferably, the mean pore diameter of the non-fibrous porous film is 0.3 to 10 µm.

Preferably, the polymer binder of the adhesion layer is a water soluble polymer or an organic solvent soluble polymer.

In another aspect, the invention provides a method for producing the multilayer analytical element according to any one of claims 1 to 9, which comprises coating at least one adhesion layer on one side of a water-non- transmitting planar support, and then laminating a non-fibrous porous film whose bending fracture strength is 20 g weight or more and whose elongation ratio when pulled with a 50-g weight is 2% or less.

Preferably, the adhesion layer is dissolved partly by an aqueous solution, an organic solvent or an aqueous solution containing these which can dissolve the polymer binder of the adhesion layer, and then the non-fibrous porous film can be laminated.

Preferably, the adhesion layer is caused to become swelled by adding more aqueous solution, organic solvent, or aqueous solution containing these than the weight of the adhesion layer polymer binder.

Preferably, the adhesion layer is heated to a temperature exceeding the dissolving temperature of the polymer binder of the adhesion layer, upon or immediately before laminating the non-fibrous porous film.

### BEST MODE FOR CARRYING OUT THE INVENTION

Embodiments of the invention are described in the following.

The dry multilayer analytical element for liquid sample analysis according to the invention comprises a water-non-transmitting planar support. On one side of the support, at least one adhesion layer and a porous liquid-sample-developing layer are integrally layered in this order. The porous liquid-sample-developing layer consists of a non-fibrous porous film whose bending fracture strength is 20 g weight or more and whose elongation ratio upon pulling with a 50-g weight is 2% or less. The element is characterized in that a adhesion layer polymer binder is caused to become seeped up in the non-fibrous porous film to 2 to 50 µm. The dry multilayer analytical element for liquid sample analysis according to the invention is an improvement over the analytical element comprising a non-fibrous porous film laminated on a support, the improvement relating to the less-dense portion (a defect referred to as a white-out) that is formed at the center of the non-fibrous porous film following the spotting of a liquid specimen. By using the multilayer analytical element of the invention having the above-described configuration, it becomes possible to reduce uneven coloration when the analyzed solution, such as blood, emits color in the analytical element, thus enabling highly accurate analysis.

The invention has the feature of using a non-fibrous porous film whose bending fracture strength is 20 g weight or more and whose elongation ratio upon pulling with a 50-g weight is 2% or less as the porous liquid-sample-developing layer. This feature provides the effect that no disconnection is caused in the developing layer during manufacture (in the step of bonding the developing layer and the lower layer), for example. Another effect that can be obtained is that since the elongation ratio is small, the gap volume does not change. As a result, the invention can provide a dry multilayer analytical element having a small lot-to-lot difference and a small intra-lot difference and high measurement accuracy, which can be reduced in size and which enables stabilization of the manufacturing process.

In accordance with the invention, the bending fracture strength of the non-fibrous porous film used as the porous liquid-sample-developing layer is 20 g weight or more, preferably 30 g weight or more, more preferably 50 g weight or more. The bending fracture strength of the present invention can be measured by bending a porous film measuring 2 cm (width)x5 cm (length) into a loop, placing a predetermined weight on the loop portion, and then determining the weight upon breakage and destruction of the porous film.

In accordance with the invention, the elongation ratio of the non-fibrous porous film used as the porous liquid-sample-developing layer, when pulled by a 50-g weight; is 2% or less, preferably 1% or less, more preferably 0.5% or less, particularly more preferably 0.1% or less, and most preferably 0.0%. The elongation ratio of the present invention when pulled by the 50-g weight can be measured in the following manner. The non-fibrous porous film is cut into a strip measuring 2 cm×8 cm. A piece of taping is affixed to each end of the film; one is provided with an opening. A clip is affixed to the side of the strip having no opening, and the strip is hung with the clip. After measuring the length of the strip in the no-load condition, a 50-g weight is hung on the opening and the length of the strip is measured in the loaded-condition. And then, the ratio of one to the other (i.e., the length of the strip in the non-loaded condition to the length of the strip in the loaded condition) is calculated.

In the dry multilayer analytical element of the invention, a adhesion layer polymer binder is caused to become seeped up in the non-fibrous porous film to 2 to 50 µm. Preferably, the adhesion layer binder is caused to become seeped up in the non-fibrous porous film to 2 to 40 µm, more preferably 7 to 30 µm.

The dry multilayer analytical element of the invention is manufactured by laminating a non-fibrous porous film.

In accordance with the invention, the adhesion layer polymer binder is caused to seep up in the non-fibrous porous film to 1 µm or more in the following way. Namely, a solution (dampening water) containing a solvent that dissolves the polymer binder of the adhesion layer is coated on the adhesion layer, or the adhesion layer is impregnated with the solvent. The adhesion layer is further heated, if necessary, to cause the polymer binder to swell and increase its adhesion, followed by the laminating of the non-fibrous porous film. The amount of the dampening water is 16 g/m² or more; the heater temperature is above the dissolving temperature of the polymer. Preferably, the amount of the dampening water is 16 g/m² or more.

Alternatively, a polymer having a dissolving temperature lower than the laminating temperature may be used as the adhesion layer polymer binder, which is heated if necessary, followed by the laminating of the film without the dampening water.

The dampening water is used to partially dissolve or expand the adhesion layer polymer binder. The solvent may be water, organic solvent, or a mixture thereof, in which surface active agent, hardener, polymer binder, and the like are dissolved. Examples of the organic solvent include low-boiling-temperature-alcohols, such as methanol, ethanol, isopropyl alcohol, n-propyl alcohol, and butyl alcohol, and ketones such as acetone, and methyl ethyl ketone. Preferable examples are methanol, ethanol, isopropyl alcohol, n-propyl alcohol, and acetone. These may be used either individually, in the form of a mixture of the organic solvents, or in the form of a mixture with water.

The amount of the dampening water added is adjusted depending on the solubility of the polymer binder and the dampening water so as to adjust the adhesion of the adhesion layer polymer binder. The amount of the dampening water added is 1% to 1000% with respect to the amount of the polymer binder; preferably, it is 50 to 500%; and more preferably, it is 100% to 300%. However, if the adhesion layer polymer binder is adhesive, the dampening water may not be used.

As the water-non-transmitting planar support, a conventional water-non-transmitting support used in conventional dry analytical elements can be used. For example, it may be a film- or sheet-like support made of a polymer, such as polyethylene terephthalate, bisphenol A polycarbonate, polystyrene, cellulose ester (such as cellulose diacetate, cellulose triacetate, and cellulose acetate propionate, for example), with a thickness ranging from about 50 µm to about 1 mm, and preferably from about 80 µm to about 300 µm. The support may be either transparent or nontransparent.

If necessary, the bonding between the support and the functional layer provided thereon may be strengthened by providing an underlayer on the surface of the support. Alternatively, instead of the underlayer, the bonding may be strengthened by subjecting the surface of the support to a physical or chemical activation process.

The dry multilayer analytical element of the invention comprises a porous liquid-sample-developing layer comprising at least one non-fibrous porous film. The porous liquid-sample-developing layer is a layer with the function of spreading a component in an aqueous specimen in a planar fashion without substantially causing the component to be unevenly distributed, so that the component can be supplied to the functional layer at a substantially constant ratio per unit area.

The number of porous liquid-sample-developing layers is not limited to one; it may comprise a laminate of two or more layers of non-fibrous porous films bonded by an adhesive that is partially located. The porous liquid-sample-developing layer may also include a spread-control agent, such as a hydrophilic polymer, in order to control its spreading property. Further, a reagent for causing a desired detection reaction, a reagent for promoting the detection reaction, a variety of reagents for reducing or preventing an interfering or blocking reaction, or some of these reagents may be contained.

The porous liquid-sample-developing layer of the invention comprises a non-fibrous porous film. Preferably, the non-fibrous porous film is a porous film made of an organic polymer, which film may be either symmetric or asymmetric. In the case of an asymmetric porous film, the asymmetry ratio is preferably 2.0 or more. In the case of a symmetric porous film, the asymmetry ratio is preferably not more than 2.0. The asymmetric porous film herein refers to a porous film having a larger mean diameter of pores on one surface than that on the other surface. The asymmetry ratio refers to the value obtained by dividing the larger mean pore diameter with the smaller mean pore diameter. In the case of an asymmetric porous film, it is preferable to provide the surface of the developing layer with the larger mean pore diameter.

Preferable examples of the porous film made of an organic polymer include: 6, 6-nylon; 6-nylon; acrylate copolymer; polyacrylate; polyacrylonitrile; polyacrylonitrile copolymer; polyamide, polyimide; polyamide-imide; polyurethane; polyether sulfone; polysulfone; a mixture of polyether sulfone and polysulfone; polyester; polyester carbonate; polyethylene; polyethylene chlorotrifluoroethylene copolymer; polyethylene tetrafluoroethylene copolymer; polyvinyl chloride; polyolefin; polycarbonate; polytetrafluoroethylene; polyvinylidene difluoride; polyphenylene sulfide; polyphenylene oxide; polyfluorocarbonate; polypropylene; polybenzoimidazole; polymethyl methacrylate; styrene-acrylonitrile copolymer; styrene-butadiene copolymer; a saponified substance of ethylene-vinyl acetate copolymer; polyvinyl alcohol; and a mixture thereof.

Of these, more preferable are: 6, 6-nylon; 6-nylon; polyether sulfone; polysulfone; a mixture of polyether sulfone and polysulfone; polyethylene; polypropylene; polyolefin; polyacrylonitrile; polyvinyl alcohol; polycarbonate; polyester carbonate; polyphenylene oxide; polyamide; polyimide; polyamide-imide; and a mixture thereof.

More preferable examples are polysulfone, polyether sulfone, 6,6-nylon, and 6-nylon; particularly more preferable examples are polysulfone and polyether sulfone; a most preferable example is polysulfone.

The thickness of the non-fibrous porous film is preferably 80 to 300 µm; more preferably it is 100 to 200 µm; particularly preferably it is 130 to 160 µm.

The mean pore diameter of the non-fibrous porous film is preferably 0.3 to 10 µm; more preferably it is 0.45 to 5 µm.

In one example (1) of the dry multilayer analytical element for liquid sample analysis according to the invention, one or a plurality of adhesion layers are disposed on the transparent support, and further a porous liquid-sample-developing layer is disposed on the adhesion layer. In another example (2), one or a plurality of adhesion layers are disposed on the transparent support, and further, on the adhesion layer, there is disposed a porous liquid-sample-developing layer that contains a reagent for sample analysis. Thus, the porous liquid-sample-developing layer of the invention may or may not contain a reagent for sample analysis.

In the case of the porous liquid-sample-developing layer containing a reagent, a porous film may be immersed in a reagent solution and then dried so as to produce a reagent-containing film. In another method, the porous film may be coated with a reagent solution, which is then dried so as to produce a reagent-containing non-fibrous porous film; the method, however, is not particularly limited.

The dry multilayer analytical element of the invention includes at least one adhesion layer. The number of the adhesion layers is not particularly limited; it may be one or two or more, for example. The adhesion layer has the function of binder the porous film with the support. By bonding the porous film with a support having high strength, the film is provided with mechanical strength and with processing suitability. When the adhesion layer has other functions (such as water-absorption or coloration), such bonding also provides liquid communication between the developing layer and the adhesion layer.

The polymer binder used in the adhesion layer may be a hydrophilic polymer binder or an organic-solvent-soluble polymer binder. The hydrophilic polymer binder may comprise a natural polymer, such as gelatin, agarose, or dextran, or any of the other wide variety of substances, such as polyvinyl alcohol, polyacrylamide, polyacrylic acid, and polyvinyl pyrrolidone, as disclosed in JP Patent Publication (Kokoku) No. 1-33782 B (1989). For the organic-solvent-soluble polymer binder, a wide variety of substances, such as polyvinymethyl ether, polyvinyl butyral, or polyvinyl pyrrolidone, may be used.

The adhesion layer may be provided with other functions, such as: a water-absorbing layer for absorbing a liquid reagent; a mordant layer for preventing the diffusion of dye produced by chemical reaction; a gas transmitting layer for selectively transmitting gas; an intermediate layer for suppressing or promoting the transfer of substance between layers; an elimination layer for eliminating endogenous substance; a light-shielding layer for enabling a stable reflective photometry; a color shielding layer for suppressing the influence of an endogenous dye; a separation layer for separating blood cells and plasma; a reaction (reagent) layer containing a reagent that reacts with a target of analysis; and a coloring layer containing a coloring agent. These functional layers may be present between the support and the adhesion layer.

The thickness of the adhesion layer ranges from 0.1 µm to 1 mm; preferably from 1 µm to 100 µm; more preferably from 2 µm to 50 µm.

In the following, the various functional layers that can be included in the adhesion layer are described.

### (Reagent layer)

The reagent layer is a water-absorbing and water-permeable layer comprising a hydrophilic polymer binder in which at least some of a reagent composition that reacts with a detected component in an aqueous liquid to produce an optically detectable change is substantially uniformly dispersed. The reagent layer includes an indicator layer and a coloring layer.

A hydrophilic polymer that can be used as the polymer binder in the reagent layer is generally a natural or synthetic hydrophilic polymer with a swelling rate ranging from about 150% to about 2000%, and preferably from about 250% to about 1500%, at 30°C, upon water absorption. Examples of such a hydrophilic polymer include: gelatin (such as acid-treated gelatin or deionized gelatin, for example) disclosed in JP Patent Publication (Kokai) No. 60-108753 A (1985); a gelatin derivative (such as phthalated gelatin or hydroxyacrylate graft gelatin, for example); agarose; pullulan; pullulan derivative; polyacrylamide; polyvinyl alcohol; and polyvinylpyrrolidone.

The reagent layer may be a layer appropriately cross-linked and cured using a crosslinking agent. Examples of the crosslinking agent include: for gelatin, known vinylsulfon crosslinking agent, such as 1, 2-bis(vinylsulfonyl acetoamide)ethane and bis(vinylsulfonylmethyl)ether, and aldehydes; and, for methallyl alcohol copolymer, aldehydes and epoxy compounds containing two glycidyl groups and the like.

The thickness of the reagent layer when dried is preferably in the range of about 1 µm to about 100 µm, and more preferably about 3 µm to about 30 µm. Preferably, the reagent layer is substantially transparent.

The reagent contained in the reagent layer or other layers in the dry multilayer analytical element of the invention may be appropriately selected depending on the tested substance to be detected.

For example, when analyzing ammonia (in cases where the tested substance is ammonia or ammonia-producing substance), examples of a coloring ammonia indicator include: leuco dyes, such as leucocyanine dye, nitro-substituted leuco. dye, and leucophthalein dye (see U.S. Patent No. Re. 30267 or JP Patent Publication (Kokoku) No. 58-19062 B (1983); pH indicators, such as bromophenol blue, bromocresol green, bromthymol blue, quinoline blue, and rosolic acid (see Encyclopaedia Chimica, Vol. 10, pp 63-65, published by Kyoritsu Shuppan K. K.); triarylmethane dye precursors; leucobenzylidene dyes (see JP Patent Publication (Kokai) Nos. 55-379 A (1980) and 56-145273 A (1981)); diazonium salt and azo dye couplers; and base bleaching dyes. The content of the coloring ammonia indicator with respect to the weight of the polymer binder is preferably in the range of about 1 to about 20% by weight.

The reagent that reacts with an ammonia-producing substance as a tested substance to produce ammonia is preferably an enzyme or a reagent that contains an enzyme; the enzyme suitable for analysis may be selected appropriately depending on the type of the ammonia-producing substance as the tested substance. When an enzyme is used as the regent, the combination of the ammonia-producing substance and the reagent is determined by the specificity of the enzyme. Examples of the combination of the ammonia-producing substance and an enzyme as the reagent include: urea/urease; creatinine/creatinine deiminase; amino acid/amino-acid dehydrogenase; amino acid/amino-acid oxidase; amino acid/ammonia lyase; amine/amine oxidase; diamine/amine oxidase; glucose and phosphoamidate/phosphoamidate-hexose phosphotransferase; ADP/carbamate kinase and carbamoyl phosphate; acid amide/amide hydrolase; nucleobase/nucleobase deaminase; nucleoside/nucleoside deaminase; and nucleotide/nucleotide deaminase; guanine/guanase. An alkaline buffer that can be used in the reagent layer during the analysis of ammonia may be a buffer with a pH of 7.0 to 12.0, and preferably 7.5 to 11.5.

In addition to the reagent that reacts with an ammonia-producing substance to produce ammonia, an alkaline buffer, and a hydrophilic polymer binder with a film-forming capability, the reagent layer for the analysis of ammonia may include a wetting agent, a polymer binder crosslinking agent (curing agent), a stabilizing agent, a heavy-metal ion trapping agent (complexing agent), and the like, as needed. The heavy-metal ion trapping agent is used for masking heavy-metal ions that hinder enzyme activity. Examples of the heavy-metal ion trapping agent include complexanes such as: EDTA·2Na; EDTA·4Na; nitrilotriacetic acid (NTA); and diethylenetriaminepentaacetic acid.

Examples of the glucoses-measuring reagent composition include glucose oxidase, peroxidase, 4-aminoantipyrine or derivatives thereof, and an improved Trinder's reagent composition including 1,7-dihydroxynaphthalene, as described in U.S. Patent No. 3,992,158, JP Patent Publication (Kokai) Nos. 54-26793 A (1979), 59-20853 A (1984), 59-46854 A (1984), and 59-54962 A (1984).

### (Light-shielding layer)

A light-shielding layer may be provided on top of the reagent layer as needed. The light-shielding layer is a water-transmitting or water-permeable layer comprising a small amount of hydrophilic polymer binder with a film-forming capability in which particles with light-absorbing or light-reflecting property (together referred to as "light-shielding property") are dispersed. The light-shielding layer blocks the color of the aqueous liquid supplied to the developing layer (to be described later) by spotting, particularly the color red of hemoglobin in the case where the sample is whole blood, when measuring detectable changes (in color or in coloration, for example) that developed in the reagent layer by reflection photometry from the light-transmitting support side. In addition, the light-shielding layer also functions as a light-reflecting layer or a background layer.

Examples of the particle with light-reflecting property include: titanium dioxide particles (microcrystalline particles of rutile type, anatase type, or brookite type, with a particle diameter of about 0.1 µm to about 1.2 µm); barium sulfate particles; aluminum particles; and microflakes. Examples of the light-absorbing particles include: carbon black, gas black, and carbon microbeads, of which titanium dioxide particles and barium sulfate particles are preferable. Particularly, anatase-type titanium dioxide particles are preferable.

Examples of the hydrophilic polymer binder with a film-forming ability include regenerated cellulose of weak hydrophilicity and cellulose acetate, in addition to hydrophilic polymers similar to the hydrophilic polymer used for the manufacture of the aforementioned reagent layer. Of these, gelatin, gelatin derivatives, and polyacrylamide are preferable. Gelatin or gelatin derivatives may be used in a mixture with a known curing agent (crosslinking agent).

The light-shielding layer may be provided by applying an aqueous dispersion of light-shielding particles and a hydrophilic polymer onto the reagent layer by a known method and then drying. Alternatively, instead of providing the light-shielding layer, a light-shielding particle may be contained in the aforementioned developing layer.

### (Adhesion layer)

A adhesion layer may be provided on top of the reagent layer in order to bond and stack the developing layer, via a layer such as a light-shielding layer as needed.

The adhesion layer is preferably made of a hydrophilic polymer such that the adhesion layer is capable of bonding the developing layer when moistened or swollen with water, so that the individual layers can be integrated. Examples of the hydrophilic polymer that can be used for the manufacture of the adhesion layer are hydrophilic polymers similar to those hydrophilic polymers used for the manufacture of the reagent layer. Of these, gelatin, gelatin derivatives, and polyacrylamide are preferable. The dried-film thickness of the adhesion layer is generally 0.1 µm to 1 mm, preferably 1 µm to 100 µm, and particularly preferably 2 µm to 50 µm.

The adhesion layer may be provided on any desired layer other than the reagent layer for improving the adhesion between other layers. The adhesion layer may be provided by applying an aqueous solution of a hydrophilic polymer and, as needed, a surface active agent or the like onto the support or the reagent layer by a known method, for example.

### (Water-absorbing layer)

The dry multilayer analytical element of the invention may be provided with a water-absorbing layer between the support and the reagent layer. The water-absorbing layer is a layer consisting primarily of a hydrophilic polymer that becomes swollen by absorbing water, so that it can absorb water in the aqueous liquid sample that has reached or permeated the boundary of the water-absorbing layer. The water-absorbing layer functions to promote the permeation of blood plasma, which is the aqueous liquid component in the case where the sample is whole blood, to the reagent layer. The hydrophilic polymer used in the water-absorbing layer may be selected from those used in the aforementioned reagent layer. For the water-absorbing layer, gelatin, gelatin derivatives, polyacrylamide, and polyvinyl alcohol are generally preferable. Particularly, the aforementioned gelatin and deionized gelatin are preferable. Most particularly, the aforementioned gelatin used in the reagent layer is preferable. The thickness of the water-absorbing layer when dried is about 3 µm to about 100 µm, preferably about 5 µm to about 30 µm. The amount of coating is about 3 g/m² to about 100 g/m², and preferably about 5 g/m² to about 30 g/m². The pH of the water-absorbing layer upon use (during the implementation of analysis operation) may be adjusted by adding a pH buffer or a known basic polymer or the like in the water-absorbing layer, as will be described later. The water-absorbing layer may further contain a known dye mordant or a polymer dye mordant, for example.

### (Detection layer)

The detection layer is generally a layer in which a dye or the like produced in the presence of a detected component is diffused and becomes optically detectable through a light-transmitting support. The detection layer may consist of a hydrophilic polymer, and it may contain a dye mordant, such as a cationic polymer for an anionic dye, for example. The water-absorbing layer generally refers to a layer in which the dye produced in the presence of the detected component is not substantially diffused, and it is distinguished from the detection layer in this respect.

The reagent layer, water-absorbing layer, adhesion layer, developing layer and the like may each contain a surface active agent, of which one example is a nonionic surface active agent. Examples of nonionic surface active agent include: p-octylphenoxypolyethoxyethanol, p-nonylphenoxypolyethoxyethanol, polyoxyethylene oleyl ether, polyoxyethylene sorbitan monolaurate, p-nonylphenoxypolyglycidol, and octyl glucoside. By having the nonionic surface active agent contained in the developing layer, its function of spreading the aqueous liquid sample (metering function) can be improved. By having the nonionic surface active agent contained in the reagent layer or the water-absorbing layer, the water in the aqueous liquid sample can be facilitated to be substantially uniformly absorbed by the reagent layer or the water-absorbing layer during analysis operation, so that the contact of the liquid with the developing layer can take place quickly and substantially uniformly.

The tested substance that can be analyzed by the dry multilayer analytical element of the invention is not particularly limited and a particular component in any liquid sample (including bodily fluids, such as whole blood, blood plasma, blood serum, lymph fluid, urine, saliva, cerebrospinal fluid, and vaginal fluid; drinking water, liquors, river water, and factory waste water) can be analyzed. For example, the dry multilayer analytical element can be used for the analysis of albumin (ALB), glucose, urea, bilirubin, cholesterol, proteins, enzymes (including blood enzymes such as lactic dehydrogenase, CPK (creatine kinase), ALT (alanineamino-transferase), AST (aspartate aminotransferase), and GGT (γ-glutamyltranspeptidase)).

The dry multilayer analytical element of the invention can be prepared by known methods. Hemolysis reagent may be added in the reagent solution in advance for application or impregnation. In another method, the developing layer may be coated with an aqueous solution, an organic solvent (ethanol or methanol, for example), or a solution of a water-organic solvent mixture, either alone or containing a surface active agent or a hydrophilic polymer for spread area control, so as to impregnate the developing layer with the hemolysis reagent. The tested substance may be analyzed using this method in accordance with a known method.

For example, the dry multilayer analytical element of the invention may be cut into small pieces of squares with each side measuring about 5 mm to about 30 mm, or circles of similar sizes. They can then be accommodated in a slide frame such as described in JP Patent Publication (Kokoku) No. 57-283331 B (1982) (corresponding to U.S. Patent No. 4169751), JP Utility Model Publication (Kokai) No. 56-142454 U (1981) (corresponding to U.S. Patent No. 4387990), JP Patent Publication (Kokai) No. 57-63452 A (1982), JP Utility Model Publication (Kokai) No. 58-32350 U (1983), and JP Patent Publication (Kohyo) No. 58-501144 A (1983) (corresponding to WO083/00391), and the slide can then be used as a chemical analytical slide. This is preferable from the viewpoint of manufacture, packaging, shipping, storage, measurement operation, and so on. Depending on the purpose of use, the element may be stored in a cassette or a magazine in the form of an elongated tape. Alternatively, such small pieces may be stored in a container with an opening, they may be affixed to or accommodated in an opening card, or the cut pieces may be used as is.

In the dry multilayer analytical element of the invention, about 2 µL to about 30 µL, and preferably 4 µL to 15 µL of an aqueous liquid sample is spotted on the porous liquid-sample-developing layer. The thus spotted dry multilayer analytical element is then incubated at a certain temperature ranging from about 20°C to about 45°C, preferably from about 30°C to about 40°C, for 1 to 10 minutes. The coloration or change in color in the dry multilayer analytical element is measured from the developing layer side (when the support is non-transparent) or from any one of developing layer or support side (when the support is transparent) by reflection photometry, and the amount of the tested substance in the specimen can be determined using a prepared analytical curve based on the principle of colorimetry.

A highly accurate quantitative analysis can be performed by a very simple procedure using a chemical analyzer such as those disclosed in JP Patent Publication (Kokai) Nos. 60-125543 A (1985), 60-220862 A (1985), 61-294367 A (1986), 58-161867 A (1983) (corresponding to U.S. Patent No. 4,424,191), for example. Depending on the purpose or the desired level of accuracy, the degree of coloration may be judges visually and a semi-quantitative analysis may be performed.

Since the dry multilayer analytical element of the invention is stored in a dry state until the beginning of analysis, there is no need to prepare a reagent as required. Further, as the reagents are generally more stable in a dry state, the dry multilayer analytical element of the invention can be more simply and quickly utilized than the so-called wet methods, in which solutions of reagents must be prepared as required. The invention is also superior as an examination method whereby a highly accurate examination can be performed with small quantities of liquid sample.

The invention will be hereafter described in more detail by way of examples thereof. The invention is not limited by these examples.

### Examples

### Test Example 1: Strength and elongation ratio of polysulfone film

A polysulfone film (SE-200: manufactured by Fuji Photo Film Co., Ltd., to be hereafter referred to as a PS film) was cut into a strip measuring 2 cmx5 cm. The ends of the strip were overlapped to form a loop at the center. In the loop, weights of 50, 20, and 10 g were placed quietly and left at rest for 2 to 3 seconds. Thereafter, the strip was bent in the opposite direction, and the weights were again placed on the previously bent portion and left at rest for 2 to 3 seconds. After performing this operation, the frequency of breakage of the strip was confirmed. Table 1 shows the resultant number of strips that were severed upon implementation of the present test. The result of Table 1 indicates that the PS film can remain very stable with respect to the handling during manufacture.

**Table 1. Result of evaluation of bending test**

| Weight (g/weight) | PS film |
|---|---|
| 50 | 0/5 |
| 20 | 0/5 |
| 10 | 0/5 |

The PS film was cut into a strip measuring 2 cmx8 cm, a strip of taping was affixed to both ends of the film, and an opening was provided in one of the ends. A clip was affixed to the side having no opening so as to hang the strip, and the length of the strip was measured in the no-load condition. Then, a 50-g weight was hung on the opening in order to measure the length of the strip in the loaded condition, followed by the calculation of the elongation ratio. The measurements were conducted at 22°C and humidity of 36%. Table 2 shows the elongation ratio determined from the present test. The results of Table 2 show that, under the present test conditions, the PS film exhibits no elongation at all, indicating that the PS film does not exhibit any change during manufacture, thereby reducing intra-lot difference and lot-to-lot difference.

**Table 2. Elongation ratios when pulled by a 50-g weight**

| | PS film |
|---|---|
| Length with no load (cm) | 4.98 |
| Length with load (cm) | 4.98 |
| Elongation ratio | 0.0% |

### Example 1

A adhesion layer of gelatin was coated on the 188-µm-thick PET (polyethylene terephthalate) support to the coating amount of 17 g/m². Then, on the gelatin film coated on the support, dampening water containing a hardener was coated to the coating amount of 29 g/m². Immediately after the application of the dampening water, the adhesion layer was heated with a heater with the surface temperatures of (1) 50°C, (2) 45°C, (3) 40°C, and (4) 25°C, thereby swelling the gelatin and increasing its adhesion. This was followed by the lamination of a polysulfone porous film (SE200 manufactured by Fuji Photo Film Co., Ltd.). After blow-drying, the laminate was left to stand in a constant-temperature bath so as to harden the gelatin. The hardening of gelatin provided a more uniform and stronger bonding between the gelatin layer and the polysulfone porous film. Through these steps, an analytical element comprised of the support, the adhesion layer, and the non-fibrous porous film was fabricated.

### Comparative Example 1

An analytical element was fabricated by the same method as in Example 1 with the exception that the application amount of the dampening water containing hardener was 15 g/m², and the surface temperature of the heater was 25°C.

### Comparative Example 2

An analytical element was fabricated by the same method as in Example 1 with the exception that the dampening water that contained hardener comprised a mixture solution of water and ethanol with the water-to-ethanol ratio of 1:2, and its application amount was 15 g/m², and the laminating was conducted at room temperature.

### Evaluation 1: Seeping evaluation

The above analytical elements produced in Example 1 and Comparative Examples 1 and 2 were longitudinally cut and vapor-deposited with platinum so as to prepare samples. The samples were put under an optical microscope or an SEM to obtain cross-sectional images, based on which the seeping of gelatin into the film was measured. The results are shown in Table 3. The height of seeping of gelatin in the analytical element of Comparative Example 2 was 1 µm or less; this was not suitable for actual applications because of the peeling of the film during processing. Table 3 shows the gelatin seeping height in the analytical element according to Example 1 (temperatures: 50°C, 45°C, 40°C, and 25°C), Comparative Example 1, and Comparative Example 2.

### Evaluation 2: Visual determination of unevenness in spotting

The above analytical elements produced in Example 1 and Comparative Examples were each spotted with 6 µl of an aqueous solution of dye, and then the white-out level on the spotted side and the back side was visually determined. The results are shown in Table 3.

**Table 3**

| | Amount of water added (ml/m²) | Temperature (°C) | Seeping height (µm) | Processing suitability | White-out (Visual) |
|---|---|---|---|---|---|
| Example 1(1) | 29 | 50 | 30 | Possible | - |
| Example 1(2) | 29 | 45 | 29 | Possible | - |
| Example 1(3) | 29 | 40 | 6.5 | Possible | - |
| Example 1(4) | 29 | 25 | 5.7 | Possible | A little + |
| Comparative Example 1 | 15 | 25 | 1.8 | Possible | ++ |
| Comparative Example 2 | 5 | 25 | <1 | Impossible (film peeling) | No evaluation |

| | | | | | |
|---|---|---|---|---|---|
| Notes: +: white-out present; -: white-out absent | | | | | |

### Evaluation 3: Measurement of uneven coloration using a chromatoscanner

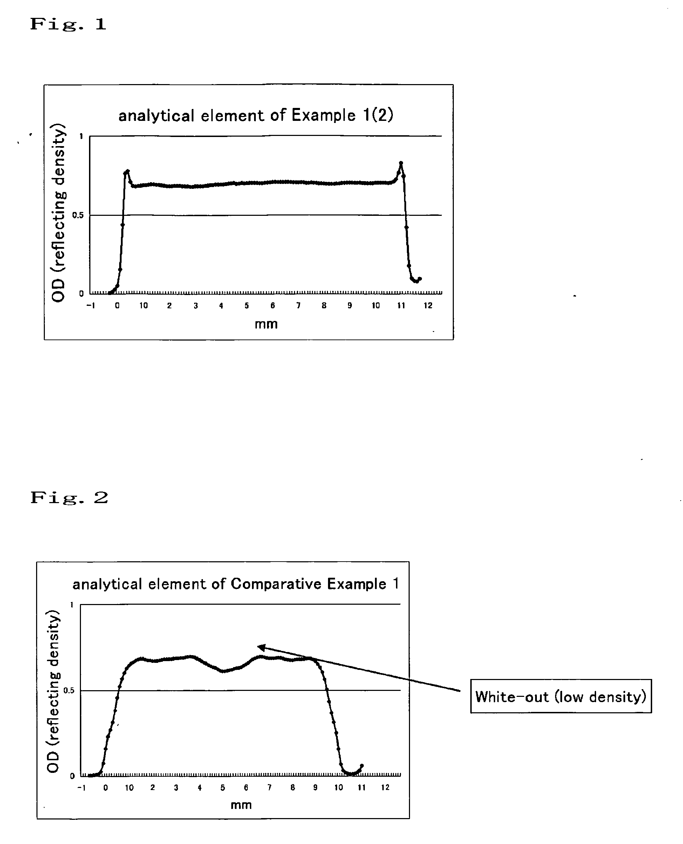

The analytical elements produced in Example 1 (2) and Comparative Example 1 were each spotted with 6 µl of aqueous solution of dye, and they were then subjected, without placing in a mount, to the measurement of coloration density unevenness, using a chromatoscanner (CS-9300PC densitometer by Shimadzu Corporation). They were also placed in a plastic mount and processed therein, and then subjected to measurement, using a medical specimen examining apparatus DRI-CHEM5000 by Fuji Film Co., Ltd. The spotting liquid consisted of a simulated liquid of 1% polyvinyl pyrrolidone aqueous solution in which red dye [0.8 mg/ml] was dissolved. The analytical element produced in Comparative Example 2 was unable to be formed into an integral analytical element; and therefore it could not be evaluated in terms of the spotting of the dye-containing aqueous solution. After the analytical elements of Example 1 and Comparative Example 1 were each spotted with the dye, they were horizontally scanned with a densitometer CS-9300PC by Shimadzu Corporation in order to measure the dye density distribution, thereby observing the dye condition in the gelatin layer (Figs. 1 and 2).

In the analytical element produced in Comparative Example, a portion with less density was observed at the center following the spotting of the dye solution (Fig. 2), which is herein referred to as a "white-out." Such white-out, which indicates the variations in reflecting densities, adversely affects measurement accuracy. On the other hand, Fig. 1 shows the result of spotting the same dye solution on the analytical element produced in Example 1. It can be clearly seen that the white-out is eliminated and that the density is uniform, indicating the uniform seeping. The uniform seeping of dye stabilized the optical density upon reflective photometry and led to improved analysis accuracy.

### Example 2

A multilayer analytical element was fabricated using the amount of the aqueous solution added of 29 g/m² and the heater temperature of 45°C among the fabricating conditions of Example 1, and using the non-fibrous porous films shown in Table 4. The table also shows the results of visual inspection of uneven coloration upon spotting of the dye solution as described in Example 1. The results showed that there was no white-out in any of the analytical elements in which the adhesion layer was caused to become greatly seeped up according to this fabricating process.

**Table 4**

| Non-fibrous porous film | Film thickness | Pore diameter | White-out (visual) |
|---|---|---|---|
| Polyether sulfone film | 115 | 5 | - |
| Cellulose acetate film | 150 | 5 | - |
| Cellulose acetate film | 135 | 3 | - |
| Cellulose acetate film | 125 | 0.45 | - |
| Nylon film | 170 | 0.45 | - |

| | | | |
|---|---|---|---|
| Notes: +: white-out present; -: white-out absent | | | |

### INDUSTRIAL APPLICABILITY

In the dry multilayer analytical element of the invention, a non-fibrous porous film whose bending fracture strength is 20 g weight or more and whose elongation ratio upon pulling by a 50-g weight is 2% or less is used as the developing layer. In this way, the effect can be obtained that the breakage of the developing layer during manufacture (in the step of bonding the developing layer and the lower layer) does not occur. Another effect provided by the invention is that since the elongation ratio is low, the gap volume does not change. Thus, the invention provides a dry multilayer analytical element in which the lot-to-lot difference and the intra-lot difference are small, which has high measurement accuracy, which can be reduced in size, and which contributes to the stabilization of the manufacturing process. Further, in the dry multilayer analytical element of the invention, the non-fibrous porous film has the adhesion layer polymer binder seeped up therein to 2 to 50 µm. In this way, the white-out can be eliminated and measurement accuracy can be improved. Thus, the present invention provides a multilayer analytical element that has sufficient processing strength and high measurement accuracy.

### BRIEF DESCRIPTION OF THE DRAWINGS

Fig. 1 shows the results of measurement using the analytical element of Example 1.
Fig. 2 shows the results of measurement using the analytical element of Comparative Example.

## Claims

1. A dry multilayer analytical element for the analysis of a liquid sample which comprises a water-non-transmitting planar support on one side of which at least one adhesion layer and a porous liquid-sample-developing layer are integrally layered in the mentioned order, wherein the porous liquid-sample-developing layer comprises a non-fibrous porous film whose bending fracture strength is 20 g weight or more and whose elongation ratio when pulled with a 50-g weight is 2% or less, and wherein a adhesion layer polymer binder is caused to become seeped up in the non-fibrous porous film to 2 to 50 µm.

2. The multilayer analytical element according to claim 1 wherein the adhesion layer binder is caused to become seeped up in the non-fibrous porous film to 2 to 40 µm.

3. The multilayer analytical element according to claim 1 or 2 wherein the adhesion layer binder is caused to become seeped up in the non-fibrous porous film to 7 to 30 µm.

4. The multilayer analytical element according to any of claims 1 to 3 wherein the non-fibrous porous film is 6, 6-nylon; 6-nylon; acrylate copolymer; polyacrylate; polyacrylonitrile; polyacrylonitrile copolymer; polyamide, polyimide; polyamide-imide; polyurethane; polyether sulfone; polysulfone; a mixture of polyether sulfone and polysulfone; polyester; polyester carbonate; polyethylene; polyethylene chlorotrifluoroethylene copolymer; polyethylene tetrafluoroethylene copolymer; polyvinyl chloride; polyolefin; polycarbonate; polytetrafluoroethylene; polyvinylidene difluoride; polyphenylene sulfide; polyphenylene oxide; polyfluorocarbonate; polypropylene; polybenzoimidazole; polymethyl methacrylate; styrene-acrylonitrile copolymer; styrene-butadiene copolymer; a saponified substance of ethylene-vinyl acetate copolymer; polyvinyl alcohol; or a mixture thereof.

5. The multilayer analytical element according to any of claims 1 to 4 wherein the non-fibrous porous film is polysulfone, polyether sulfone, 6,6-nylon, or 6-nylon.

6. The multilayer analytical element according to any of claims 1 to 5 wherein the non-fibrous porous film is an asymmetric film.

7. The multilayer analytical element according to any of claims 1 to 6 wherein the film thickness of the non-fibrous porous film is 80 to 300 µm.

8. The multilayer analytical element according to any of claims 1 to 7 wherein the mean pore diameter of the non-fibrous porous film is 0.3 to 10 µm.

9. The multilayer analytical element according to any of claims 1 to 8 wherein the binder of the adhesion layer is a water soluble polymer or an organic solvent soluble polymer.

10. A method for producing the multilayer analytical element according to any one of claims 1 to 9, which comprises coating at least one adhesion layer on one side of a water-non-transmitting planar support, and then laminating a non-fibrous porous film whose bending fracture strength is 20 g weight or more and whose elongation ratio when pulled with a 50-g weight is 2% or less.

11. The method according to claim 10, wherein the adhesion layer is dissolved partly by an aqueous solution, an organic solvent or an aqueous solution containing these which can dissolve the polymer binder of the adhesion layer, and then the non-fibrous porous film can be laminated.

12. The method according to claim 10 or 11, wherein the adhesion layer is caused to become swelled by adding more aqueous solution, organic solvent, or aqueous solution containing these than the weight of the adhesion layer polymer binder.

13. The method according to any of claims 10 to 12, wherein the adhesion layer is heated to a temperature exceeding the dissolving temperature of the polymer binder of the adhesion layer, upon or immediately before laminating the non-fibrous porous film.
